# EUROPEAN PATENT APPLICATION

(11) **EP 1 820 459 A2**
(43) Date of publication of application: **22.08.2007**
(21) Application number: 07250599.3
(22) Date of filing: 14.02.2007
(51) Int. Cl.: A61B 17/32

(54) **A method for sealing a blood vessel, a medical system and a medical instrument**

(30) Priority: 15.02.2006 US 354372
(71) Applicant: ETHICON ENDO-SURGERY, INC., Cincinnati, Ohio 45242 (US)
(72) Inventor: Messerly, Jeffrey D., Cincinnati, Ohio 45244-4116 (US); Neuenfeldt, Steven K., Pleasanton, CA 94566 (US)
(74) Representative: Tunstall, Christopher Stephen

(57) **Abstract**

A medical instrument includes a medical end effector and a user-actuated media transporter. The medical end effector includes an ultrasound propagating element and includes a path adapted for directing a medical agent, when conveyed therealong, to the ultrasound propagating element. The user-actuated media transporter is adapted for conveying the medical agent along the path and into contact with the ultrasound propagating element. A medical system includes a medical instrument and a user-actuated hemostatic-agent transporter. The medical instrument is adapted for treating patient tissue and is a mechanical-based ligation instrument or an energy-based ligation instrument. The user-actuated hemostatic-agent transporter is adapted for conveying a hemostatic agent to the patient tissue. A method for sealing a blood vessel of a patient includes applying a hemostatic agent to the blood vessel and includes treating the blood vessel with a medical instrument which is a mechanical-based ligation instrument or an energy-based ligation instrument.

## Description

**Field of the Invention**

The present invention is related generally to surgical instruments and to surgical methods, and more particularly to: a method for sealing a blood vessel of a patient; to a medical system including a mechanical-based or an energy-based ligation instrument such as an ultrasonic surgical shears, a clip applier, a stapler, and an RF (radio-frequency) bipolar vessel sealer; and to a medical instrument having a medical end effector including an ultrasound propagating element.

**Background of the Invention**

A conventional ultrasonic surgical shears includes an end effector having an ultrasonic surgical blade and a clamping arm operable to open and close toward the blade, wherein the ultrasonic surgical blade is adapted for vibrating at a frequency in the range of 20 kilohertz to 500 kilohertz. In one known application, the ultrasonic surgical shears is used as an energy-based ligation instrument for transecting and sealing a blood vessel, or other tissue, of a patient. Other conventional ligation instruments include a clip applier, a stapler, and an RF (radio-frequency) bipolar vessel sealer. Known medical agents include hemostatic agents such as coagulum, other therapeutic agents such as medicines, and tissue-imaging-enhancing material such as a tissue dye for improved radiographic imaging. Medical syringes are known for applying a liquid to patient tissue.

Still, scientists and engineers continue to seek improved medical instruments which have a medical end effector including an ultrasound propagating element, improved medical systems which include a mechanical-based or an energy-based ligation instrument, and improved methods for sealing a blood vessel of a patient.

**Summary of the Invention**

A first embodiment of the invention is for a medical instrument including a medical end effector and a user-actuated media transporter. The medical end effector includes an ultrasound propagating element and includes a path adapted for directing a medical agent, when conveyed therealong, to the ultrasound propagating element. The user-actuated media transporter is adapted for conveying the medical agent along the path and into contact with the ultrasound propagating element.

A second embodiment of the invention is for a medical system including a medical instrument and a user-actuated hemostatic-agent transporter. The medical instrument is adapted for treating patient tissue and is chosen from the group consisting of a mechanical-based ligation instrument and an energy-based ligation instrument. The user-actuated hemostatic-agent transporter is adapted for conveying a hemostatic agent to the patient tissue.

A method of the invention is for sealing a blood vessel of a patient. The method includes applying a hemostatic agent to the blood vessel. The method includes treating the blood vessel with a medical instrument chosen from the group consisting of a mechanical-based ligation instrument and an energy-based ligation instrument.

Several benefits and advantages are obtained from one or more of the method and the embodiments of the invention. In one example, the medical agent has a more viscous state when conveyed (without being ultrasonically vibrated) via mechanical translation, mechanical rotation, mechanical translation with rotation, fluidic pressure differentials, et cetera along the path and has a less viscous state when ultrasonically vibrated by the ultrasound propagating element allowing for improved dispersal of the medical agent. In another example, conveying a hemostatic agent to patient tissue, such as applying the hemostatic agent to a blood vessel, improves hemostasis when the patient tissue, such as a blood vessel, is treated with a mechanical-based or energy-based ligation instrument.

The present invention has, without limitation, application in hand-activated instruments as well as in robotic-assisted instruments.

**Brief Description of the Figures**

FIGURE 1 is a schematic side-elevational view of a first embodiment of the invention wherein the medical instrument includes a channel type of path in the medical end effector (which is shown in cross section) for conveying therein a medical agent to the ultrasound propagating element of the medical end effector and includes a syringe for conveying the medical agent in the channel;

FIGURE 2 is a view, as in Figure 1, but of an alternate embodiment of the medical instrument of Figure 1 (with the outer sheath of the end effector omitted for clarity) including a different channel type of path and a different ultrasound propagating element;

FIGURE 3 is a schematic cross-sectional view of the end effector of another alternate embodiment of the medical instrument of Figure 1 including a rod type of path and a different ultrasound propagating element;

FIGURE 4 is a side-elevational view of cross sectional view of a second embodiment of the invention wherein the medical system includes an ultrasonic surgical shears and includes a mister adapted for spraying a hemostatic agent on patient tissue; and

FIGURE 5 is a perspective view of the ultrasonic surgical shears of Figure 4 and of a hemostatic agent which includes a sleeve having coagulum and which is adapted to be carried on a prong of the ultrasonic surgical shears.

**Detailed Description of the Invention**

Before explaining the present invention in detail, it should be noted that the invention is not limited in its application or use to the details of construction and arrangement of parts illustrated in the accompanying drawings and description. The illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.

It is understood that any one or more of the following-described embodiments, examples, et cetera can be combined with any one or more of the other following-described embodiments, examples, et cetera.

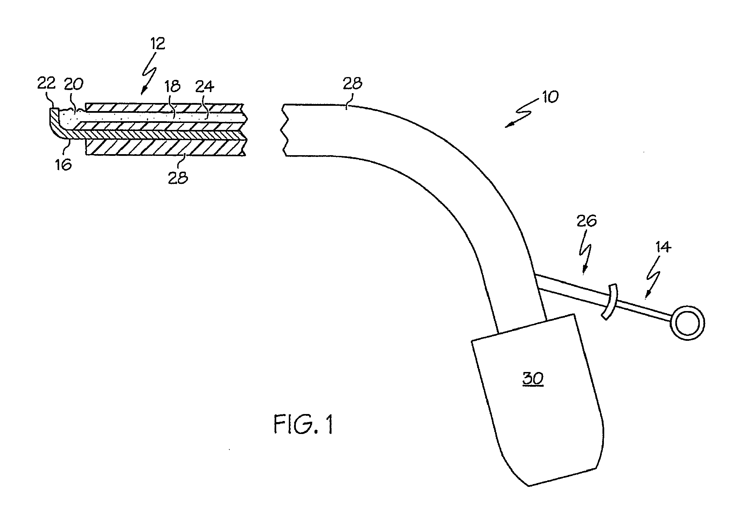

Referring now to the Figures, in which like numerals indicate like elements, Figure 1 illustrates a first embodiment of the invention. A first expression of the embodiment of Figure 1 is for a medical instrument 10 including a medical end effector 12 and a user-actuated media transporter 14. The medical end effector 12 includes an ultrasound propagating element 16 and includes a path 18 adapted for directing a medical agent 20, when conveyed therealong, to the ultrasound propagating element 16. The user-actuated media transporter 14 is adapted for conveying the medical agent 20 along the path 18 and into contact with the ultrasound propagating element 16.

In one enablement of the first expression of the embodiment of Figure 1, the medical agent 20 has a more viscous state (including, without limitation, the solid state and a mix of solid and liquid / gaseous states such as pellets in a viscous liquid carrier or stabilized microbubbles in a solid polymeric carrier) when conveyed (without being ultrasonically vibrated) along the path 18 and has a less viscous state when ultrasonically vibrated by the ultrasound propagating element 16. In one employment, improved dispersal of the medical agent 20 is achieved, and the dispersed medical agent returns to the more viscous state, staying in place, when it is no longer being ultrasonically vibrated by the ultrasound propagating element 16.

An ultrasound propagating element 16 is an element adapted for vibrating at ultrasonic frequencies. In one utilization, the ultrasound propagating element 16 is adapted to vibrate at a frequency in the range of 20 kilohertz to 500 kilohertz. In one example, the ultrasound propagating element 16 has a distal-most vibration antinode 22 and the media transporter 14 is adapted for conveying the medical agent 20 along the path 18 and in contact with an area of the ultrasound propagating element 16 at or near its distal-most vibration antinode 22. In one application, the ultrasound propagating element 16 is a curved ultrasonic blade. The ultrasound propagating element 16 delivers thermal energy to the medical agent 20. This energy delivery can occur quickly to the medical agent 20 and can occur in close proximity to the contract area (i.e. energy deposition can be characterized by a steep thermal gradient) thus providing control of energy delivery to the medical agent 20. With this controlled energy delivery, the medical agent 20 can be delivered in a controlled manner; the user can initiate and stop delivery of the medical agent 20 in rapid succession.

Examples of medical agents include, without limitation, therapeutic agents which have a medical effect on patient tissue and imaging-enhancing agents which improve visibility of the medical agent and adjacent tissue (such as is necessary for marking tissue sites and identifying boundaries for clinical follow-up) when the site is viewed with a medical imaging device. Examples of medical imaging devices include, without limitation, diagnostic ultrasound, magnetic resonance imaging, computed tomography imaging, and radiographic imaging devices. Examples of tissue-imaging-enhancing agents include, without limitation, radioisotopes, radiographic dyes, microbubbles, iron particles, gadolinium chelates, manganese chelates, et cetera. In one application, a therapeutic agent has a medically active portion and a medically inactive portion, wherein the medically inactive portion is chosen such that the medical agent has a more viscous state when conveyed along the path and a less viscous state when ultrasonically vibrated by the ultrasound propagating element. Once in place, the medical agent cools until it reaches thermal equilibrium with the surrounding tissue, thus returning the medical agent to a more viscous state (such as, without limitation, the solid state). Imaging of the medical agent and adjacent tissue is improved by virtue of at least one of the following: a) the aforementioned enhancing agents present mismatched and differentiated boundaries with the inactive portion of the medical agent or tissue that the imaging modalities listed above are known to resolve; and b) the cooled medical agent itself presents a mismatched and differentiated boundary with adjacent tissue that, again, the imaging modalities listed above are known to resolve.

Examples of therapeutic agents include, without limitation, polymers, glues, cements, and drugs that individually or as a combined agent provide clinical effects such as having a procoagulative (via tamponade or chemically induced such as by clot promotion), cell death, growth inhibition, cell growth / transplantation, ablation, bulking, infection inhibition, pain relief, and/or approximation (such as by adhesively bonding) effect. In one variation, the therapeutic agent includes a biodegradable material that is absorbed by the patient tissue over a period of time. This biodegradable agent can be a solid polymer or viscous fluid and can include bound drugs, gene therapies or viable biological entities that, by virtue of the extended time frame for local absorption (versus delivered alone in a less viscous state), provide for a controlled or long lasting clinical effect. In the same or a different variation, the therapeutic agent includes an adhesive material that approximates or joins structures such as devices, implants or patient tissue. In the same or a different variation, the therapeutic agent has a medically active state and a medically inactive state, wherein the medical agent is made medically active by external means such as ultrasonic pressure waves or light.

In an implementation of the first expression of the embodiment of Figure 1, the path 18 includes a channel 24, and the user-actuated media transporter 14 is adapted for conveying the medical agent 20 in the channel 24. In the same or a different implementation, the medical agent 20 has a more viscous liquid state when conveyed (without being ultrasonically vibrated) along the path 18 and has a less viscous liquid state when ultrasonically vibrated by the ultrasound propagating element 16. In one variation, the path 18 includes a channel 24 and the user-actuated media transporter 14 includes a syringe 26. In one example, the medical end effector 12 includes an outer sheath 28, the channel 24 is a lumen of the outer sheath 28, the ultrasound propagating element 16 extends or is extendable from the outer sheath 28, the medical instrument 10 includes a handpiece 30, and the handpiece 30 and the syringe 26 are connected to the outer sheath 28.

Other shapes of the ultrasound propagating element, not shown, include: those having a hole through which the medical agent 20 passes through as it contacts the wall surrounding the hole; those having a split element (one curved up, the other curved down) and two paths (one directing a first medical agent to the curved-up element and another directing a second medical agent to the curved-down element); those having a single element, with an upper end pin and a lower end pin, and two paths (one directing a medical agent to the upper end pin and another directing a medical agent to the lower end pin); and those whose ultrasound propagating element is proximal a distal needle carried by an outer tube having a sidewall cutout exposing the element.

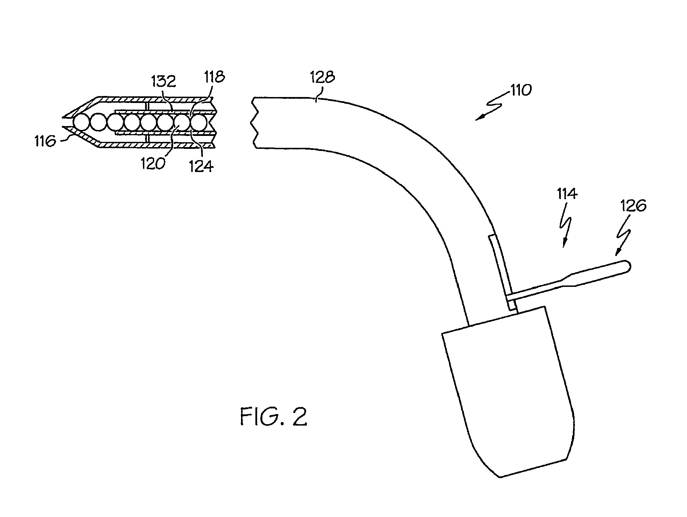

A first alternate embodiment of the medical instrument 110 is shown in Figure 2. In one enablement, the medical agent 120 has a solid state (or a mix of solid and liquid states such as pellets in a liquid carrier) when conveyed (without being ultrasonically vibrated) along the path 118 and has a liquid state when ultrasonically vibrated by the ultrasound propagating element 116. In one variation, the user-actuated media transporter 114 includes a push slide 126 that extends along the device to force the medical agent 120 distally and into contact with the ultrasound propagating element 116. As shown in Figure 2, the push side 126 provides force to the media by way of sliding or translatory transmission from the user. Similarly, axial force and/or rotational torque may be provided to the medical agent 120 by, for example, a lead screw. In one modification, the ultrasound propagating element 116 has a needle shape and is coaxially aligned with the channel 124. In one example, the medical agent 120, before being ultrasonically vibrated, includes a series of solid medical agents each having a spherical shape. Other examples of shapes of solid medical agents, not shown, include, without limitation, a series of cylinders having flat ends and a series of cylinders having ball-and-socket ends. In one construction, the channel 124 is a channel of an inner sheath 132 which may be vibrationally isolated from the ultrasound propagating element 116 at vibration nodes via, for example, ribs, o-rings, et cetera.

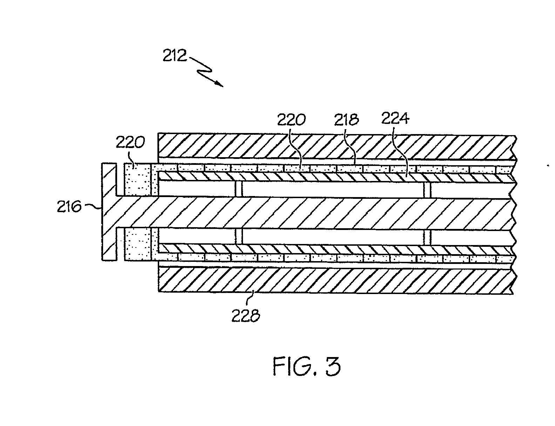

A second alternate embodiment of the medical instrument 210 is shown in Figure 3. In one enablement, the path 218 includes a rod 224 that is tubular in shape, and the medical agent 220 has an annular shape and is adapted to be installed on the rod 224. In this enablement, the user-actuated media transporter (not shown, but in one example is an annular push slide otherwise identical to the push slide 126 shown in Figure 2 and in another example is a lead screw for imparting translation and/or rotation to the medical agent 220) is adapted for conveying the medical agent 220 along the rod 224 and in contact with the distal end (e.g., a ball or needle shaped distal end) of the ultrasound propagating element 216. In one example, the medical agent 220, before being ultrasonically vibrated, includes a series of solid medical agents each having a ring shape. Other examples of annular shapes, not shown, include, without limitation, a series of tubes. In one construction, the rod 224 is an inner sheath rod which surrounds and may be connected to the ultrasound propagating element 216 at vibration nodes via, for example, ribs, o-rings, et cetera so that structural support is provided with minimal impact on the vibrational performance of the ultrasound propagating element 216 (i.e., the rod 224 is vibrationally isolated from the ultrasound propagating element 216.

A method for medically treating patient tissue using the medical instrument 10 of the first expression of the embodiment of Figure 1, wherein the medical agent 20 becomes less viscous when ultrasonically vibrated, includes steps a) through c). Step a) includes disposing the medical end effector 12 proximate the patient tissue to be medically treated. Step b) includes using the user-actuated media transporter 14 to convey the medical agent 20 in the more viscous state (such as, without limitation, the solid state) along the path 18 and in contact with the ultrasound propagating element 16. Step c) includes activating the ultrasound propagating element 16 to thermally change the medical agent 20 to the less viscous state to disperse the medical agent 20. Step d) includes deactivating the ultrasound propagating element 16 to thermally return the dispersed medical agent 20 to the more viscous state (such as, without limitation, the solid state).

In one employment, the medical agent 20 has at least one effect chosen from the group consisting of tissue marking, tissue site imaging enhancement, coagulation via tamponade, coagulation via chemically induced clot promotion, cell death, tissue growth inhibition, tissue ablation, tissue bulking, infection inhibition, pain relief, cell growth / transplantation, approximation of tissues, approximation of devices, and approximation of implants. In the same or a different employment, the user-actuated media transporter includes a lead screw. In the same or a different employment, there is also included coating the medical agent 20 with a coating material to reduce sticking of the medical agent 20 to the ultrasound propagating element 16. Examples of coating materials include, without limitation, Teflon suspensions, Paralene, MDX (a silicone dispersion), and titanium nitride.

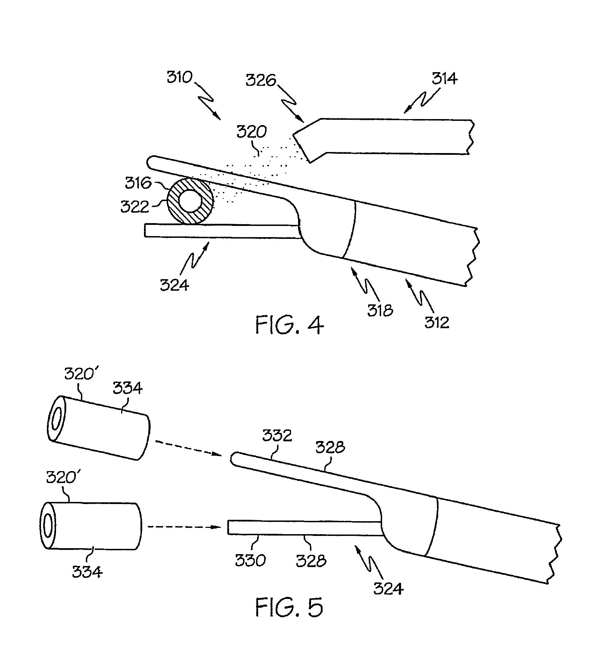

Referring again to the Figures, Figure 4-5 illustrate a second embodiment of the invention. A first expression of the embodiment of Figures 4-5 is for a medical system 310 including a medical instrument 312 and a user-actuated hemostatic-agent transporter 314. It is noted that the term "user" includes, without limitation, a human user and a robot user. The medical instrument 312 is adapted for treating patient tissue 316 and is chosen from the group consisting of a mechanical-based ligation instrument and an energy-based ligation instrument 318. The user-actuated hemostatic-agent transporter 314 is adapted for conveying a hemostatic agent 320 to the patient tissue 316.

In one enablement of the first expression of the embodiment of Figures 4-5, the patient tissue 316 includes a blood vessel 322. In one variation, the medical instrument 312 is an energy-based ligation instrument 318 adapted to transect the blood vessel 322. In one modification, the energy-based ligation instrument 318 includes an ultrasonic surgical shears 324. Other examples of energy-based ligation instruments include, without limitation, a bipolar vessel sealer. Examples of mechanical-based ligation instruments include, without limitation, clip appliers and tissue staplers. Examples of hemostatic agents 320 include, without limitation, adhesives such as glues (e.g., cynoacrylites), adhesives such as epoxies (e.g., urethanes), etc.

In one application of the first expression of the embodiment of Figures 4-5, the user-actuated hemostatic-agent transporter 314 includes a mister 326 adapted for spraying the hemostatic agent 320 on the patient tissue 316. Other user-actuated hemostatic-agent transporters include, without limitation, the previously-described media transporters 14.

In one implementation of the first expression of the embodiment of Figures 4-5, the hemostatic agent 320 has hemostatic properties when activated by energy, and the medical instrument 312 is an energy-based ligation instrument 318 adapted to activate the hemostatic properties of the hemostatic agent 320. In one variation, the hemostatic agent 320 includes a protein adapted to be denatured creating coagulum by energy from the energy-based ligation instrument 318.

A method of the invention is for sealing a blood vessel 322 of a patient. The method includes applying a hemostatic agent 320 to the blood vessel 322. The method includes treating the blood vessel 322 with a medical instrument 312 chosen from the group consisting of a mechanical-based ligation instrument and an energy-based ligation instrument 318.

In one implementation of the method, the medical instrument 312 is the energy-based ligation instrument 318, and the energy-based ligation instrument 318 is chosen from the group consisting of an ultrasonic surgical shears 324 and a bipolar vessel sealer. In one variation, the hemostatic agent 320 includes a protein adapted to be denatured creating coagulum by energy from the energy-based ligation instrument 318. In the same or a different variation, the energy-based ligation instrument 318 is an ultrasonic surgical shears 324 having a prong 328 (either the ultrasonic blade 330 or the clamping arm 332 of the ultrasonic surgical shears 324) and the hemostatic agent 320 includes a sleeve 334 (e.g., the hemostatic agent has an annular shape) which includes coagulum and is adapted to be carried on the prong 328 (two sleeves 330 and two prongs 328 are shown in Figure 5). In a different implementation of the method, the medical instrument 312 is the mechanical-based ligation instrument and the mechanical-based ligation instrument is chosen from the group consisting of a clip applier and a stapler.

Several benefits and advantages are obtained from one or more of the method and the embodiments of the invention. In one example, the medical agent has a more viscous state when conveyed (without being ultrasonically vibrated) via mechanical translation, mechanical rotation, mechanical translation with rotation, fluidic pressure differentials, et cetera along the path and has a less viscous state when ultrasonically vibrated by the ultrasound propagating element allowing for improved dispersal of the medical agent. In another example, conveying a hemostatic agent to patient tissue, such as applying the hemostatic agent to a blood vessel, improves hemostasis when the patient tissue, such as a blood vessel, is treated with a mechanical-based or energy-based ligation instrument.

While the present invention has been illustrated by a description of several embodiments and a method, it is not the intention of the applicants to restrict or limit the spirit and scope of the appended claims to such detail. Numerous other variations, changes, and substitutions will occur to those skilled in the art without departing from the scope of the invention. For instance, the medical instrument and the medical system of the invention have application in robotic assisted surgery taking into account the obvious modifications of such systems, components and methods to be compatible with such a robotic system. It will be understood that the foregoing description is provided by way of example, and that other modifications may occur to those skilled in the art without departing from the scope and spirit of the appended Claims.

## Claims

1. A medical instrument comprising:
a) a medical end effector including an ultrasound propagating element and including a path adapted for directing a medical agent, when conveyed therealong, to the ultrasound propagating element; and
b) a user-actuated media transporter adapted for conveying the medical agent along the path and into contact with the ultrasound propagating element.

2. The medical instrument of claim 1, also including the medical agent, wherein the medical agent has a more viscous state when conveyed along the path and has a less viscous state when ultrasonically vibrated by the ultrasound propagating element.

3. The medical instrument of claim 2, wherein the medical agent has a more viscous liquid state when conveyed along the path and has a less viscous liquid state when ultrasonically vibrated by the ultrasound propagating element.

4. The medical instrument of claim 3, wherein the path includes a channel and wherein the user-actuated media transporter includes a syringe.

5. The medical instrument of claim 2, wherein the medical agent has a solid state when conveyed along the path and has a liquid state when ultrasonically vibrated by the ultrasound propagating element.

6. The medical instrument of claim 5, wherein the user-actuated media transporter includes a push slide.

7. The medical instrument of claim 2, wherein the ultrasound propagating element is adapted to vibrate at a frequency in the range of 20 kilohertz to 500 kilohertz.

8. The medical instrument of claim 2, wherein the path includes a channel and wherein the user-actuated media transporter is adapted for conveying the medical agent in the channel.

9. The medical instrument of claim 2, wherein the path includes a rod that is tubular in shape, wherein the medical agent has an annular shape and is adapted to be installed on the rod, and wherein the user-actuated media transporter is adapted for conveying the medical agent along the rod.

10. A medical system comprising:
a) a medical instrument adapted for treating patient tissue and chosen from the group consisting of a mechanical-based ligation instrument and an energy-based ligation instrument; and
b) a user-actuated hemostatic-agent transporter adapted for conveying a hemostatic agent to the patient tissue.

11. The medical system of claim 10, wherein the patient tissue includes a blood vessel, wherein the medical instrument is an energy-based ligation instrument adapted to transect the blood vessel, and wherein the energy-based ligation instrument includes an ultrasonic surgical shears.

12. The medical system of claim 10, wherein the user-actuated hemostatic-agent transporter includes a mister adapted for spraying the hemostatic agent on the patient tissue.

13. The medical system of claim 10, wherein the hemostatic agent has hemostatic properties when activated by energy and wherein the medical instrument is an energy-based ligation instrument adapted to activate the hemostatic properties of the hemostatic agent.

14. The medical system of claim 13, wherein the hemostatic agent includes a protein adapted to be denatured creating coagulum by energy from the energy-based ligation instrument.
